**Europäisches Patentamt**

**European Patent Office**

**Office européen des brevets**

(19)

(11) Veröffentlichungsnummer: **0 159 679**
**A2**

(12) # EUROPÄISCHE PATENTANMELDUNG

(21) Anmeldenummer: **85104793.6**

(22) Anmeldetag: **20.04.85**

(51) Int. Cl.⁴: **A 61 F 13/06**

(30) Priorität: **27.04.84 DE 3415657**

(43) Veröffentlichungstag der Anmeldung: **30.10.85**
**Patentblatt 85/44**

(84) Benannte Vertragsstaaten: **AT BE CH FR GB IT LI NL SE**

(71) Anmelder: **Thum, Oskar, Martinistrasse 52,**
**D-2000 Hamburg 20 (DE)**
Anmelder: **von Torklus, Detlef, Prof. Dr.,**
**Jungfrauenthal 20, D-2000 Hamburg 13 (DE)**

(72) Erfinder: **Thum, Oskar, Martinistrasse 52,**
**D-2000 Hamburg 20 (DE)**
Erfinder: **von Torklus, Detlef, Prof. Dr.,**
**Jungfrauenthal 20, D-2000 Hamburg 13 (DE)**

(74) Vertreter: **Minetti, Ralf, Dipl.-Ing., Ballindamm 15,**
**D-2000 Hamburg 1 (DE)**

(54) **Fussgelenkbandage.**

(57) Gegenstand der Neuentwicklung bildet eine Fussgelenkbandage mit einer abgewinkelten, rohrförmigen Knöchelsocke aus einem elastischen Material wie beispielsweise einem Gummigewebe. Derartige Bandagen werden benutzt, um ein Ausknicken oder Umknicken des Fusses im Bereich des Knöchels zu verhindern. Bekannt sind weiterhin Bandagen, die aus einem Band bestehen, das um den Fuss herum gewickelt wird. Solche Bandagen können sich beim Tragen leicht lockern und lassen dadurch in ihrer Wirkung nach. Die neuartige Bandage besteht sowohl aus einer Socke wie auch aus einem mit der Socke verbundenen Zugband, welches sich in seinem unteren Abschnitt an der Seite des Fusses von der Ferse (8) bis etwa zum Vorderfuss erstreckt. Ein derartiges Zugband lässt sich gegebenenfalls nach einer vorausgehenden vollständigen Umschlingung des Vorderfusses von der Aussenseite des Fusses (11) her über den Aussenknöchel (9) hinweg spiralförmig über den Fuss um den Unterschenkel herumschlingen und beispielsweise mittels eines Klettenbandabschnittes (3) im Bereich des Unterschenkels befestigen. Eine zusätzliche Abstützung wird erreicht, wenn in der Knöchelsocke (1) im Bereich der Fussohle ein keilförmig sich erhöhendes Polster eingearbeitet ist, wobei auch das Zugband (2) mit einem zusätzlichen Stützpolster (10) für den Aussenknöchelbereich versehen sein kann.

DIPL.-ING **RALF MINETTI**

P A T E N T A N W A L T

0159679

2 HAMBURG 1, den 16. April 1985
Ballindamm 15 10/44

Fernsprecher 33 51 15

Bank: Commerzbank AG, Konto-Nr. 38/57 554
(BLZ 200 400 00)

Postscheck: Hamburg 2509 00-207
(BLZ 200 100 20)

Anm.: Thum, von Torklus, Hamburg

meine Akte: EPA 1021/85

## <u>Fußgelenkbandage</u>

Die Erfindung betrifft eine Fußgelenkbandage mit einer abgewinkelten, rohrförmigen Knöchelsocke aus elastischem Material wie Gummigewebe. Derartige Bandagen sind üblich, um ein Ausknicken bzw. Umknicken des Fußes nach außen zu verhindern und die Bänder in einer entspannten Lage zu halten. In der Praxis haben sich derartige Knöchelsocken aus elastischem Material zwar aufgrund ihrer einfachen Handhabung und ihres festen Sitzes als vorteilhaft gezeigt, andererseits aber nicht als allgemein ausreichend für einen Schutz der Bänder bei höheren Belastungen.

Anstelle der Anwendung einer elastischen Knöchelsocke ist es für die Heilung von Bänderzerrungen und Bänderdehnungen infolge des Ausknickens des Fußes bekannt, am Fuß eine Bandage anzulegen, die auch mit einer endseitig angeordneten Schlaufe zum Durchstecken des Vorderfußes versehen sein kann (DE-OS 31 22 463). Wird bei einer solchen Bandage das Band von außen nach innen um den Fuß herumgewickelt, um die Außenseite des Fußes anzuheben und dadurch die Bänder zu entlasten, so setzt das Anlegen einer derartigen Bandage eine bei einem unkundigen Benutzer nicht zu erwartende Fachkunde voraus, wenn in diesem Sinne die Bandage wirksam sein soll.

Aufgabe der Erfindung ist es, eine Fußgelenkbandage zu schaffen, die ohne fachmännische Vorkenntnisse vom Benutzer leicht zu handhaben ist und die Vorteile bekannter Bandagen miteinander verbindet. Die Erfindung sieht dafür die Verwendung einer Knöchelsocke vor, mit der ein Zugband aus elastischem Material verbunden ist, das sich im Fußsohlenbereich von der Ferse bis zum vorderen Randbereich der Knöchelsocke erstreckt und von der Außenseite des Fußes über den Außenknöchel spiralförmig über den Fuß hinweg und danach um den Unterschenkel herum verläuft.

Eine derartige Fußgelenkbandage, die auch von Benutzern ohne Vorkenntnisse leicht zu handhaben ist, eröffnet einen großen Anwendungsbereich, da sie geeignet ist für die Behandlung von Distersionen des oberen und unteren Sprunggelenkes, von Bandrupturen sowie Bandzerrungen und Banddehnungen des Außenknöchels, zur post-

operativen Nachbehandlung nach wiederherstellenden Operationen; bei primärer Hypermobilität
(Bandlaxheit) mit rezidivierenden Distorisionen
zur Vermeidung von Umknicktraumen sowie zur
Prophylaxe bei gefährdeten Athletischen Sportarten wie Basketball. Das ist darauf zurückzuführen, daß der elastische Spiralzug die beiden
Systeme Fuß und Unterschenkel analog dem Gesamtverlauf der anatomischen Bandverbindungen am
Außenknöchel verbindet. Es ergibt sich dabei
eine kraftvolle Sicherung der Position durch
die Verlagerung des anatomischen Ursprungs
der Bandverbindungen vom Außenknöchel nach
innen bzw. medial bis oberhalb des Innenknöchels, so daß eine erwünschte Hebelwirkung
erreicht wird, die ein Ausknicken des Fußes
verhindert.

Die erzwungene Fußstellung läßt sich dabei
durch den Spiralzug dosierbar sichern, wobei
eine Entlastung der zerrissenen, gedehnten
oder gezerrten Bandverbindungen des Außenknöchels durch Annäherung von Ursprung und Ansatz
bewirkt wird und damit die notwendigen Voraussetzungen für eine Ausheilung der Bänder in
der Funktionsstellung geschaffen werden.

Das Zugband braucht nicht über seine volle Länge aus elastischem Material zu bestehen. Es hat sich vielmehr als ausreichend und sogar vorteilhaft gezeigt, wenn lediglich der den Fuß unmittelbar umschlingende Abschnitt des Zugbandes aus elastischem Material besteht, um den notwendigen Druck auszuüben und den festen Sitz zu gewährleisten und wenn im übrigen das Zugband vorzugsweise aus einem Klettenband besteht, das nach Umschlingung des Unterschenkels in beliebiger Länge befestigt sein kann.

Allgemein von Vorteil ist es jedoch, wenn das Zugband im Fußsohlenbereich breiter ausgebildet ist, als im Bereich über dem Fuß, so daß der Fuß über eine möglichst große Länge seitlich angehoben wird zwecks einer Entlastung der Bänder im Sinne einer Verhinderung des Ausknickens vom Gelenk. Dafür erstreckt sich deshalb das Zugband vom hinteren Rand der Ferse bis in den Mittel- oder Vorfußbereich.

Die Befestigung des Zugbandes an der Knöchelsocke kann aus herstellungstechnischen Gründen durch Nähte vorzugsweise erfolgen, wobei das

- 5 -

Zugband mit einer Naht an der Fußaußenseite und mit einer weiteren Naht unter der Fußsohle mit der Knöchelsocke verbunden sein kann.

Besonders geeignet ist die vorgeschlagene Fußgelenkbandage auch für die zusätzliche Aufnahme einer sich vorzugsweise nach außen keilförmig erhöhenden Polsterung, die das Anheben der Fußaußenseite erwirkt. Ein solches Polster kann aus Siliconschaum bestehen und wird vorzugsweise von dem Zugband überdeckt.

Die neue Fußgelenkbandage erlaubt weiterhin die Anordnung eines Stützpolsters im Außenknöchelbereich und über dem Vorderteil des oberen Sprunggelenkes. Dieses Stützpolster kann im Zugband eingearbeitet sein. Durch die Elastizität des Zugbandes ließe sich die Einstellung des Stützpolsters zum Fuß ausrichten. Das gleiche gilt auch für eine Anordnung des keilförmigen Polsters unter der Fußsohle in dem Zugband. Als zweckmäßiger hat es sich jedoch gezeigt, wenn für eine feste gleichbleibende Stellung der Polster diese unmittelbar in der Socke eingearbeitet sind.

Wenn vorstehend und nachstehend davon gesprochen ist, daß die Bandage besonders geeignet ist, ein Ausknicken des Gelenkes bzw. des Fußes nach außen zu verhindern, so versteht es sich, daß eine im Prinzip gleichartige Bandage auch benutzt werden kann als Sicherung gegen ein Einknicken des Fußes, wobei dann eine Seitenumkehrung vorzunehmen ist hinsichtlich der Anordnung und Wicklung des Zugbandes sowie der Polster. Weiterhin fällt es mit in den Schutzbereich, beispielsweise für eine besonders starre Führung des oberen und unteren Sprunggelenkes bilateral auf beiden Seiten jeweils ein Zugband der vorgesehenen Art anzuordnen, wobei sich dann die beiden Spiralbandzüge überkreuzen.

Ein Ausführungsbeispiel der Erfindung ist nachstehend unter Bezugnahme auf eine Zeichnung erläutert. In der Zeichnung zeigen:

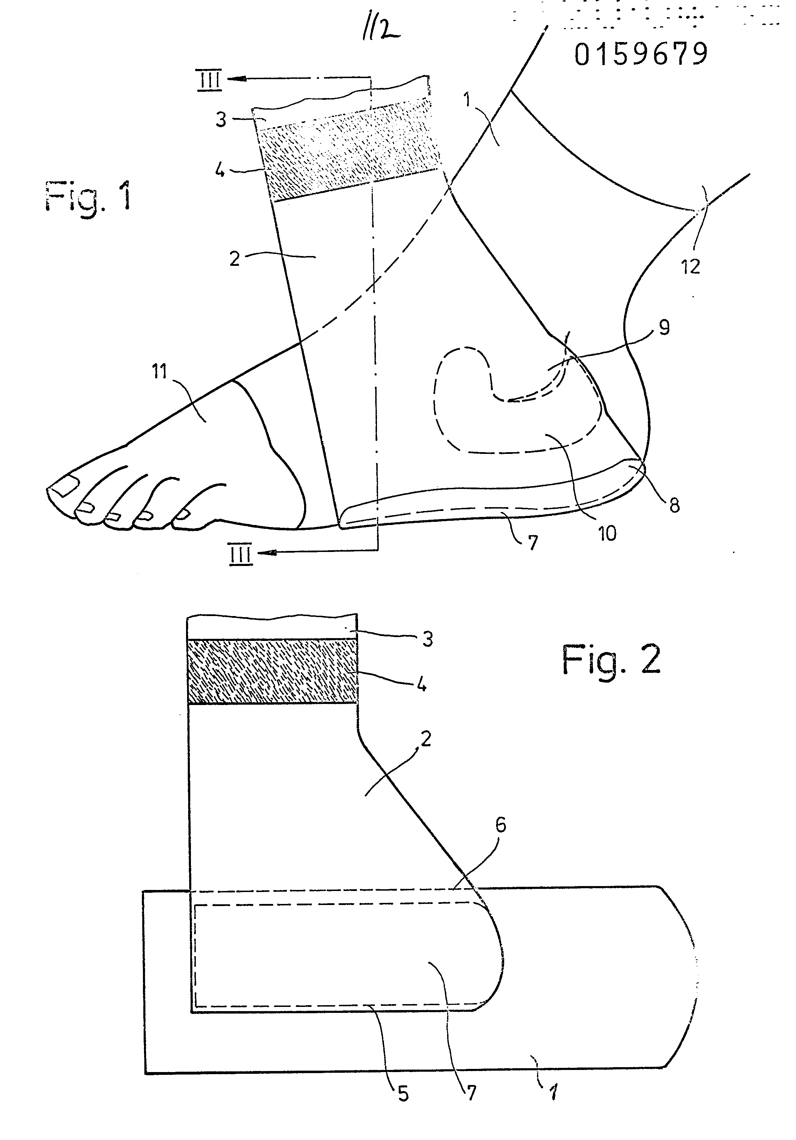

Figur 1: eine Fußgelenkbandage vor dem Anlegen des Zugbandes in der Seitenansicht;

Figur 2: die Unteransicht der Fußgelenkbandage;

Figur 3: einen Schnitt nach der Linie III- III der Figur 1 und

0159679

Figur 4: eine perspektivische Darstellung eines Fußes mit Bandage im Gebrauchszustand.

Die in der Zeichnung wiedergegebene Fußgelenkbandage besteht aus einer Knöchelsocke aus elastischem Material, nämlich einem Gummi- oder Kunststoffgewebe. Außerdem gehört zu der Gelenkbandage ein Zugband 2, dessen unterer, mit der Knöchelsocke 1 verbundener Abschnitt ebenfalls aus einem elastischen Gewebematerial besteht. An diesen unteren Abschnitt schließt sich ein Klettenband 3 an, das auf seiner Unterseite mit Haken versehen ist, mit denen das Band auf einem auf der Oberseite angeordneten Klettenverschluß 4 zu befestigen ist.

Das Zugband ist im Bereich der Seite des Fußes sich verbreiternd nach unten hin ausgebildet, so daß es bei einer Anlage am Fuß den Knöchel 9 überdeckt. Dafür erstreckt sich das Zugband 2 von der Rückseite der Ferse 8 her bis nahe zum Vorderfuß. Seine Befestigung ergibt sich aus zwei parallellaufenden Nähten 5 und 6 an der Unterseite der Knöchelsocke 1. Dadurch überdeckt das Zugband 2 ein Polster 7, das sich nach der Außenseite des Fußes hin keilför-

0159679

mig erweitert und einem Anheben der Außenseite des Fußes zwecks Entlastung des Gelenkes dient. Dieses Polster erstreckt sich ebenfalls bis zur Ferse des Fußes 11.

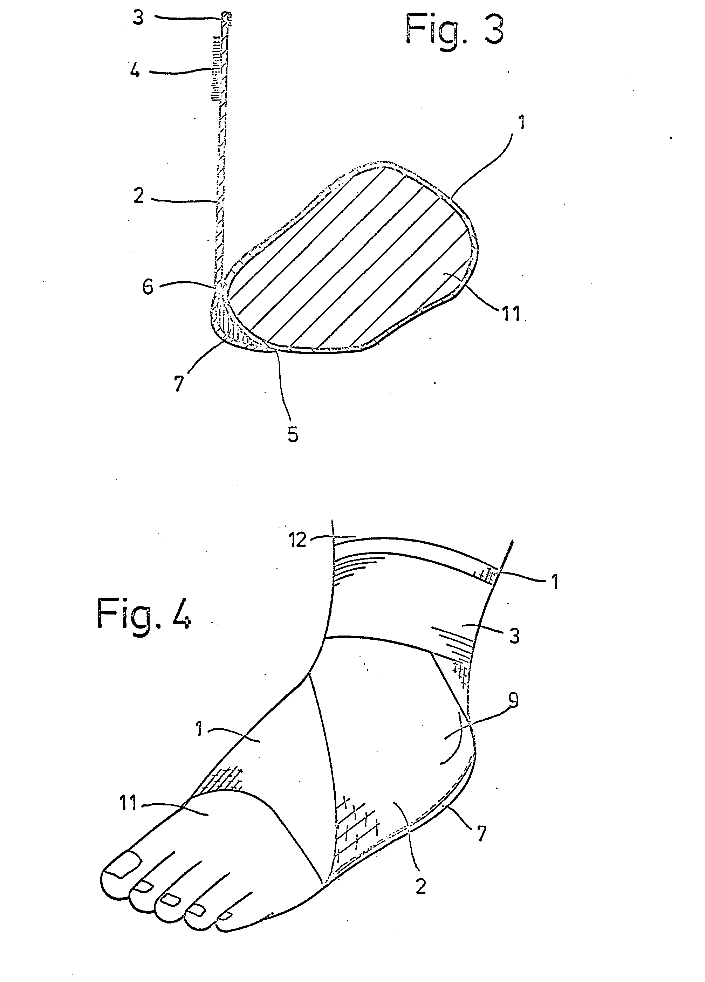

Entsprechend der Figur 4 umschlingt das Zugband den Fuß auf seiner Oberseite spiralförmig und verläuft dann in einer Schlinge um den Unterschenkel 12 herum, wobei seine wirksame Länge und damit der durch den elastischen Abschnitt auf den Fuß seitlich und von oben ausgeübte Druck eingestellt werden kann.

Zur weiteren Abstützung ist im Bereich des Knöchels 9 in der Knöchelsocke 1 ein Stützpolster 10 eingearbeitet, das den Bandzug im Außenknöchelbereich und über dem vorderen Anteil es oberen Sprunggelenkes unterpolstert und geeignet ist, beispielsweise zur Linderung von Schmerzen oder Schwellungen.

DIPL.-ING **RALF MINETTI**

P A T E N T A N W A L T

Anm.: Thum, von Torklus, Hamburg

meine Akte: EPA 1021/85

2 HAMBURG 1, den 18. April 1985

Ballindamm 15    10/44

Fernsprecher 33 51 15

Bank: Commerzbank AG, Konto-Nr. 38/57 554
(BLZ 200 400 00)

Postscheck: Hamburg 2509 00-207
(BLZ 200 100 20)

0159679

## Patentansprüche

1. Fußgelenkbandage mit einer abgewinkelten, rohrförmigen Knöchelsocke aus elastischem Material wie Gummigewebe, dadurch gekennzeichnet, daß mit der Knöchelsocke (1) ein Zugband (2) aus elastischem Material verbunden ist, das sich im Fußsohlenbereich von der Ferse (8) bis zum vorderen Randbereich der Knöchelsocke (1) erstreckt und von der Außenseite des Fußes (11) über den Außenknöchel (9) spiralförmig über den Fuß (11) hinweg und danach um den Unterschenkel herum verläuft.

2. Fußgelenkbandage nach Anspruch 1, dadurch gekennzeichnet, daß das Zugband (2) im Fußsohlenbereich breiter ausgebildet ist als im Bereich über dem Fuß (11).

3. Fußgelenkbandage nach Anspruch 1, dadurch gekennzeichnet, daß das Zugband (2) durch Nähte (5, 6) mit der Knöchelsocke (1) verbunden ist.

4. Fußgelenkbandage nach Anspruch 3, dadurch gekennzeichnet, daß das Zugband durch eine Naht (6) an der Fußaußenseite und eine weitere Naht (5) unter der Fußsohle mit der Knöchelsocke (1) verbunden ist.

5. Fußgelenkbandage nach Anspruch 1, dadurch gekennzeichnet, daß in der Knöchelsocke (1) im Fußsohlenbreich ein sich nach der Fußaußenseite keilfömig erhöhendes Polster (7) eingearbeitet ist, das vom Zugband (2) überdeckt ist.

6. Fußgelenkbandage nach Anspruch 1, dadurch gekennzeichnet, daß die Knöchelsocke (1) oder das Zugband (2) ein Stützpolster (10) im Außenknöchelbereich und/oder über dem Vorderteil des oberen Sprunggelenkes trägt.

7. Fußgelenkbandage nach Anspruch 1, dadurch gekennzeichnet, daß ein Fußsohlenpolster (7) in das Zugband (2) eingearbeitet ist.

Fig. 1

Fig. 2

0159679

2/2

0159679

Fig. 3

Fig. 4